# EUROPÄISCHE PATENTSCHRIFT

(11) **EP 0 489 185 B1**
(45) Veröffentlichungstag und Bekanntmachung des Hinweises auf die Patenterteilung: **26.06.1996**
(21) Anmeldenummer: 90123114.2
(22) Anmeldetag: 03.12.1990
(51) Int. Cl.: A61F 2/16

(54) **Intraokulare künstliche Augenlinse**
Artificial intra-ocular lens
Lentille intra-oculaire artificielle

(43) Veröffentlichungstag der Anmeldung: 10.06.1992
(73) Patentinhaber: Chiron Adatomed Pharmazeutische und Medizintechnische Gesellschaft mbH, D-85609 Dornach (DE)
(72) Erfinder: Hettlich, Hans-Joachim, Dr., W-5100 Aachen (DE); Mittermayer, Christian, Professor Dr., 52074 Aachen (DE); Klee, Doris, Dr., 52062 Aachen (DE); Richter, Horst Arnold, Dr., 52074 Aachen (DE)
(74) Vertreter: Nöth, Heinz, Dipl.-Phys.

(56) Entgegenhaltungen:
- EP-A- 0 205 997
- EP-A- 0 212 616
- WO-A-85/04566
- WO-A-89/07426
- DE-A- 3 939 648
- FR-A- 2 627 078
- US-A- 4 851 003

## Beschreibung

Die Erfindung betrifft eine intraokulare künstliche Augenlinse aus Silikongummi.

Aus der WO-A-89/07 426 ist eine intraokulare künstliche Augenlinse aus Silikongummi bekannt, die im natürlichen Kapselsack mit einem Bindemittel fixiert ist.

Die Vorteile intraokularer Augenlinsen aus Silikongummi bestehen darin, daß sie ausschließlich aus einem einzigen Material gefertigt sind, das chemisch inert und autoklavierbar ist. Ferner ist dieses Material flexibel, und mithin kann die Linse beim Implantieren auf einen relativ geringen Durchmesser verkleinert werden, so daß nur geringe Schnittöffnungen erforderlich sind. Aufgrund seiner Flexibilität nimmt der Linsenkörper seine ursprüngliche Form, insbesondere Scheibenform, im implantierten Zustand wieder an. Derartige Linsen können ohne jegliche Haptikbügel hergestellt und implantiert werden, so daß praktisch keine Penetrationsgefahr der Linsenkapsel besteht. Auch zeigt die Linse praktisch keine Fremdkörperreaktion.

Allerdings nimmt man hier in Kauf, daß sich im Kapselsack oder am Ziliarkörper keine sichere Fixation erzielen läßt und die Nachstarrate ähnlich hoch ist wie bei Polymethylmethacrylatlinsen.

Aufgabe der Erfindung ist es daher, eine intraokulare künstliche Augenlinse aus Silikongummi zu schaffen, bei der eine sichere Fixierung im natürlichen Kapselsack erreicht wird.

Diese Aufgabe wird erfindungsgemäß durch die kennzeichnenden Merkmale des Patentanspruches 1 gelöst. Die Rückseite des Linsenkörpers ist mit Proteinen beschichtet, die zur Innenseite des Kapselsackes eine biospezifische Affinität aufweisen. Diese Affinität beruht auf der Fähigkeit der Proteine, zugehörige Bestandteile der Innenseite des Kapselsackes zu erkennen und mit diesen in Wechselwirkung zu treten. Hierdurch erfolgt eine Bindung bzw. Adhäsion der an der Oberfläche des Linsenkörpers befindlichen Proteine mit der spezifisch passenden Bestandteilskomponente an der Innenseite des Kapselsackes. Es wird hiermit die biospezifische Affinität zwischen Proteinen und Bestandteilen der Innenseite des Kapselsackes ausgenützt. Auf diese Weise erreicht man eine direkte Adhäsion der implantierten künstlichen Augenlinse am Kapselsack.

Die Proteine sind durch Aktivierung der Linsenoberfläche insbesondere über funktionelle Gruppen, wie Hydroxylgruppen und/oder Carboxylgruppen, an der Linsenoberfläche immobilisiert. Bevorzugt werden diese funktionellen Gruppen durch Hydrophilisierung der Oberfläche des Linsenkörpers erreicht. Dies kann beispielsweise durch Plasmaätzung mit Sauerstoffplasma oder auch mit CO₂-Plasma geschehen. Durch Anlagerung der Proteine an die derart vorbehandelte Linsenoberfläche wird die Immobilisierung der Proteine erreicht.

Es handelt sich um Proteine, die zu Kollagen vom Typ IV eine biospezifische Affinität haben. Bevorzugt wird die Linsenoberfläche mit Fibronectin, welches kollagenbindende Domänen besitzt, behandelt. Untersuchungen haben gezeigt, daß an der Innenseite des Kapselsackes Kollagen Typ IV und auch Laminin als spezifisch passende Komponenten vorhanden sind.

Die Beschichtung erfolgt mit den Proteinen, die eine biospezifische Affinität zur Linsenkapsel aufweisen, ausschließlich an der Rückseite der intraokularen Linse. Auf diese Weise werden im Bereich der Kapsulotomie Wechselwirkungen mit den Bestandteilen des Kammerwassers ausgeschlossen. Auch bei der Hydrophilierung durch die Plasmaätzung kann daher der Bereich an der Vorderseite des Linsenkörpers ausgespart sein, der im Bereich der Kapselöffung bzw. der Kapsulotomie liegt. Es ergeben sich dann an der Linsenvorderseite zwei verschiedene Oberflächenqualitäten, nämlich im Randbereich außerhalb der Kapsulotomie ein hydrophilisierter Oberflächenbereich und ein nichthydrophilisierter Bereich, der in der Kapselöffnung an der Linsenvorderseite liegt.

Um den reaktionsträgen Silikongummi, der das Linsenmaterial bildet, beschichten zu können, werden daher zuerst funktionelle Gruppen an der Linsenoberfläche erzeugt. Dies erfolgt bevorzugt durch Plasmaätzung, wobei mit Sauerstoffplasma oder auch mit CO₂-Plasma gearbeitet werden kann. Dies führt zur Bildung von Hydroxylgruppen bzw. Carboxylgruppen an der Linsenoberfläche.

An diesen funktionellen Gruppen werden dann die Proteine, die die Affinität zur Innenseite der Linsenkapsel aufweisen, angelagert. Es handelt sich hier bevorzugt um Fibronectin. Die Beschichtung erfolgt an im Handel erhältlichen intraokularen künstlichen Augenlinsen aus Silikongummi.

Eine weitere Möglichkeit der Immobilisierung der Proteine an der Linsenoberfläche ist die kovalente Bindung der Proteine beispielsweise über Oxiranverbindungen.

Um eine Zerstörung der Linsenoberfläche bei der Plasmabehandlung im O₂-Plasma zur Erzeugung von Hydroxylgruppen bzw. im CO₂-Plasma zur Erzeugung von Carboxygruppen zu vermeiden, wird eine nach oben hin begrenzte Energie bei der Aktivierung des Gasplasmas angewendet. Als obere Energiegrenze für O₂-Plasma sind 600 W bei einer Prozeßdauer von 60 Sekunden einzuhalten.

Durch die Erfindung wird eine Fixierung der künstlichen Augenlinse in der natürlichen Linsenkapsel geschaffen. Diese bildet anatomisch wie auch optisch einen idealen Ort zur Positionierung einer Intraokularlinse nach extrakapsulärer Kataraktoperation. Man erreicht einen festen und vollständigen Verbund zwischen der implantierten Linse und der natürlichen Linsenkapsel. Hierdurch erreicht man sowohl eine einwandfreie Fixierung der Linse als auch die verringerte Gefahr von Nachstarbildung. Die bislang bei Intraokularlinsen aus Silikongummi in Kauf genommene geringe Affinität zum Kapselsack ist beseitigt. Bei der Erfindung wird dies durch polymeranaloge Reaktionen an der Oberfläche der Intraokularlinse erreicht. Durch die Vorbehandlung der Oberfläche zur Schaffung von funktionellen bzw. reaktiven Gruppen mit Hilfe der Plasmaätzung und anschließender Pfropfpolymerisation adhäsionsfördernder Proteine, die biospezifisch zu Komponenten der Innenseite der natürlichen Linsenkapsel sind, erreicht man die sichere Fixierung der implantierten Linse im Kapselsack.

## Patentansprüche

1. Intraokulare künstliche Augenlinse aus Silikongummi,
dadurch **gekennzeichnet,**
daß nur die Rückseite des Linsenkörpers mit Proteinen beschichtet ist, die zu Kollagen IV oder Laminin eine biospezifische Affinität haben und daß die Proteine durch an der Rückseite des Linsenkörpers erzeugte funktionelle Gruppen oder durch kovalente Bindung immobilisiert sind.

2. Intraokulare künstliche Augenlinse nach Anspruch 1, dadurch gekennzeichnet, daß die Rückseite des Linsenkörpers mit Fibronectin beschichtet ist.

3. Intraokulare künstliche Augenlinse nach Anspruch 1 oder 2, dadurch gekennzeichnet, daß die funktionellen Gruppen durch Hydrophilisierung der Oberfläche des Linsenkörpers gebildet sind.

4. Intraokulare künstliche Augenlinse nach einem der Ansprüche 1 bis 3, dadurch gekennzeichnet, daß die funktionellen Gruppen durch Plasmabehandlung in O₂- oder CO₂-haltigem Plasma gebildet sind.

5. Intraokulare künstliche Augenlinse nach einem der Ansprüche 1 bis 4, dadurch gekennzeichnet, daß die funktionellen Gruppen Hydroxylgruppen und/oder Carboxylgruppen sind.

6. Intraokulare künstliche Augenlinse nach Anspruch 1, dadurch gekennzeichnet, daß die Proteine kovalent über Oxiranverbindungen an die Linsenoberfläche angekoppelt sind.

## Claims

1. Intraocular artificial eye lens of silicone rubber,
characterized in that
only the posterior side of the lens body is coated with proteins that have a biospecific affinity to collagen IV or laminen, and that the proteins are immobilized by functional groups produced on the posterior side of the lens body or by covalent bonding.

2. Intraocular artificial eye lens according to claim 1, characterized in that the posterior side of the lens body is coated with fibronectin.

3. Intraocular artificial eye lens according to claim 1 or 2, characterized in that the functional groups are formed by hydrophilizing the surface of the lens body.

4. Intraocular artificial eye lens according to any one of claims 1 to 3, characterized in that the functional groups are formed by plasma treatment in O₂- or CO₂-containing plasma.

5. Intraocular artificial eye lens according to any one of claims 1 to 4, characterized in that the functional groups are hydroxyl groups and/or carboxyl groups.

6. Intraocular artificial eye lens according to claim 1, characterized in that the proteins are covalently coupled to the lens surface via oxirane compounds.

## Revendications

1. Lentille intra-oculaire artificielle en caoutchouc silicone, caractérisée par le fait que la face postérieure du corps de la lentille seulement est revêtue de protéines qui ont une affinité biospécifique pour le collagène IV ou la laminine, et que les protéines sont immobilisées par des groupes fonctionnels produits sur la face postérieure du corps de la lentille ou par liaison covalente.

2. Lentille intra-oculaire artificielle selon la revendication 1, caracterisée par le fait que la face postérieure du corps de la lentille est revêtue de fibronectine.

3. Lentille intra-oculaire artificielle selon l'une des revendications 1 et 2, caractérisée par le fait que les groupes fonctionnels sont formés par hydrophilisation de la surface du corps de la lentille.

4. Lentille intra-oculaire artificielle selon l'une des revendications 1 à 3, caractérisée par le fait que les groupes fonctionnels sont formés par traitement au plasma d'oxygène ou de CO₂.

5. Lentille intra-oculaire artificielle selon l'une des revendications 1 à 4, caractérisée par le fait que les groupes fonctionnels sont des groupes hydroxyles et/ou des groupes carboxyles.

6. Lentille intra-oculaire artificielle selon la revendication 1, caractérisée par le fait que les protéines sont fixées à la surface de la lentille par covalence par l'intermédiaire de composés oxiranniques.
